# EUROPÄISCHE PATENTANMELDUNG

(11) **EP 0 709 082 A2**
(43) Veröffentlichungstag der Anmeldung: **01.05.1996**
(21) Anmeldenummer: 95115283.4
(22) Anmeldetag: 28.09.1995
(51) Int. Cl.: A61K 7/48

(54) **Gegen unreine Haut, leichte Formen der Akne sowie Propionibacterium acnes wirksame Wirkstoffkombinationen auf der Basis von Wollwachssäuren und Glycerinestern gesättigter Fettsäuren**

(30) Priorität: 28.10.1994 DE 4438588
(71) Anmelder: Beiersdorf Aktiengesellschaft, D-20245 Hamburg (DE)
(72) Erfinder: Traupe, Bernd, D-22457 Hamburg (DE); Wolf, Florian, Dr., D-20251 Hamburg (DE); Schönrock, Uwe, Dr., D-22844 Norderstedt (DE)

(57) **Zusammenfassung**

Verwendung von Wirkstoffkombinationen aus
I) einem natürlichen Wollwachssäuregemisch oder einem durch Destillation eines natürlichen Wollwachssäuregemisches erhältlichen Wollwachssäuregemisch und
II) Monoglycerin-monocarbonsäure-monoestern
gegen unreine Haut, leichte Formen der Akne sowie Propionibacterium acnes.

## Beschreibung

Die vorliegende Erfindung betrifft Wirkstoffe und Zubereitungen, solche Wirkstoffe enthaltend, welche gegen unreine Haut wirksam sind.

Bei der unreinen Haut sind neben anderen Einflüssen bakterielle Sekundärinfektionen von ätiologischer Bedeutung. Einer der wichtigsten Mikroorganismen, der in Zusammenhang mit unreiner Haut steht, ist Propionibacterium acnes.

Unreine Haut und/oder Komedonen beeinträchtigen das Wohlbefinden der Betroffenen aber selbst in leichten Fällen. Da praktisch jeder oder jede Jugendliche von unreiner Haut irgendeiner Ausprägung betroffen ist, besteht bei vielen Personen Bedarf, diesem Zustande abzuhelfen.

Aufgabe der vorliegenden Erfindung war es also, einen gegen unreine Haut bzw. Propionibacterium acnes wirksamen Stoff zu finden.

Es wurde überraschenderweise gefunden, und darin liegt die Lösung all dieser Aufgaben, daß die Verwendung von Wirkstoffkombinationen aus
I) Einem natürlichen oder durch Destillation aufbereiteten Gemisch aus Wollwachssäuren und
II) Monoglycerin-monocarbonsäure-monoestern und
bzw. Zubereitungen, solche Wirkstoffkombinationen enthaltend, gegen unreine Haut, leichte Formen der Akne sowie Propionibacterium acnes
den Nachteilen des Standes der Technik abhilft.

Wollwachs oder Wollfett wird der bei der Rohwollwäsche anfallende fett- bis wachsartige Bestandteil der Rohschafwolle bezeichnet. Das Wollwachs besteht aus aus einem Gemisch von Fettsäureestern höherer Alkohole und aus freien Fettsäuren.

Die Hauptbestandteile der Wollwachssäuren sind
(a) gesättigte unsubstituierte Carbonsäuren, gemäß der Formel

   CH₃-(CH₂)ₙ-CH₂-COOH,
(b) α-Hydroxycarbonsäuren, gemäß der Formel 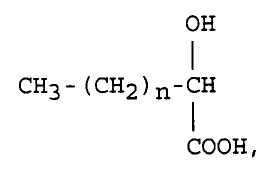
(c) ω-Hydroxycarbonsäuren, gemäß der Formel

   HO-CH₂-(CH₂)ₙ-CH₂-COOH,
(d) Isocarbonsäuren, gemäß der Formel 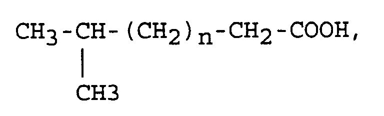
(e) α-Hydroxy-isocarbonsäuren, gemäß der Formel 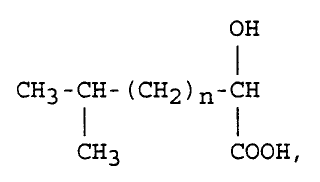
(f) ω-Hydroxy-isocarbonsäuren, gemäß der Formel 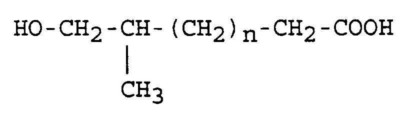
(g) Anteïsocarbonsäuren, gemäß der Formel 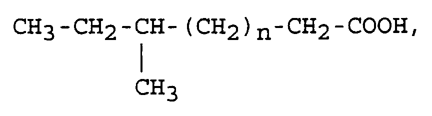
(h) α-Hydroxy-anteïsocarbonsäuren, gemäß der Formel 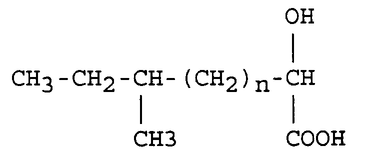
(i) ω-Hydroxy-anteïsocarbonsäuren, gemäß der Formel 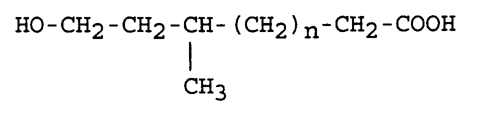
Dabei nimmt n gewöhnlich Werte von 7 - 31 an. Repräsentative Zusammensetzungen der Wollwachssäuren werden z.B. in "Parfümerie und Kosmetik", 59. Jahrgang, Nr.12/78, S.429, 430 sowie im "Lexikon der Hilfsstoffe für Pharmazie, Kosmetik und angrenzende Gebiete" von H.P.Fiedler, 1989, 3. Auflage, Editio Cantor Aulendorf, beschrieben.

Rohwollwachssäuren sind für kosmetische Zwecke nicht geeignet, statt ihrer werden für gewöhnlich destillierte Wollwachssäuren eingesetzt. Dieser Umstand und entsprechende Verfahren zur Raffinierung der Rohwollwachssäuren sind dem Fachmann bekannt.

Besonders vorteilhafte Verfahren zur Aufbereitung von Rohwollwachssäure sind beispielsweise der EP-OS 556 660 zu entnehmen.

Typischerweise bestehen Wollwachssäuren aus ca. 60 % gesättigten, unsubstituierten Carbonsäuren, ca. 30 % α-Hydroxycarbonsäuren und ca. 5 % ω-Hydroxycarbonsäuren, wobei der Rest von ca. 5 % im wesentlichen von den anderen vorgenannten Carbonsäuretypen gebildet wird.

Erfindungsgemäß besonders vorteilhaft ist eine Wollwachssäurefraktion, erhältlich durch Rohwollwachssäure durch Kurzwegdestillation im bei 10⁻¹ bar aus dem Destillationstemperaturintervall von 150 - 200° C. Der Anteil an ∝ -Hydroxycarbonsäuren beträgt dabei ca. 22 - 27 %. Solche Fraktionen zeichnen sich durch folgende kennzeichnende Parameter aus:

| | |
|---|---|
| Tropfpunkt | 50 - 54° C |
| Säurezahl | 166 - 170 |
| Verseifungszahl | 175 - 190 |
| OH - Zahl | 60 - 80 |
| Jodzahl | 7 - 12 |

Zwar ist aus dem Aufsatz "Antimicrobial Factors in Wool Wax" (Australian Journal of Chemistry, 1971, 24, Seiten 153 ff.) bekannt, daß in manchen Wollwachschargen antimikrobielle Faktoren enthalten sind. Ein Hinweis in Richtung der vorliegenden Erfindung findet sich am angegebenen Orte jedoch nicht.

Die erfindungsgemäßen Monoglycerin-monocarbonsäure-monoester (in dieser Schrift gelegentlich auch Monocarbonsäuremonoglyceride genannt) werden durch die allgemeine Formel 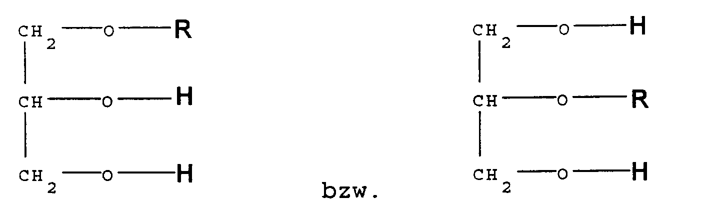
wiedergegeben, wobei R einen verzweigten oder unverzweigten Acylrest mit 6 - 14 Kohlenstoffatomen darstellt. Vorteilhaft wird R gewählt aus der Gruppe der unverzweigten Acylreste. Die diesen Estern zugrundeliegenden Fettsäuren bzw. Monocarbonsäuren sind die
Hexansäure (Capronsäure) (R = -C(O)-C₅H₁₁),
Heptansäure (Önanthsäure) (R = -C(O)-C₆H₁₃),
Octansäure (Caprylsäure) (R = -C(O)-C₇H₁₅),
Nonansäure (Pelargonsäure) (R = -C(O)-C₈H₁₇),
Decansäure (Caprinsäure) (R = -C(O)-C₉H₁₉),
Undecansäure (R = -C(O)-C₁₀H₂₁),
Dodecansäure (Laurinsäure) (R = -C(O)-C₁₁H₂₃),
Tridecansäure (R = -C(O)-C₁₂H₂₅),
Tetradecansäure (Myristinsäure) (R = -C(O)-C₁₃H₂₇).

Besonders vorteilhaft stellt R den Octanoylrest (Caprylsäurerest) bzw. den Decanoylrest (Caprinsäurerest) dar, wird also also durch die Formeln

R = -C(O)-C₇H₁₅) bzw. R = -C(O)-C₉H₁₉

repräsentiert.

In dieser Schrift, insbesondere in den Beispielen, wird das Kürzel GMCy für Glycerinmonocaprylat und das Kürzel GMC für Glycerinmonocaprinat verwendet.

Bei den in 1-Position des Glycerins veresterten Glycerinestern ist die 2-Position ein Asymmetriezentrum. Erfindungsgemäß aktiv und gleichermaßen von Vorteil sind die 2S- und die 2R-Konfiguration.

Es hat sich als günstig herausgestellt, racemische Gemische der Stereoisomeren zu verwenden.

In den erfindungsgemäßen kosmetischen bzw. dermatologischen Zubereitungen beträgt der Gehalt an GMCy und/oder GMC vorteilhaft 0,1 - 10,0 Gew.-%, bevorzugt 0,5 bis 7,5 Gew.-%, besonders bevorzugt 1,5 - 5,0 Gew.-%, jeweils bezogen auf das Gesamtgewicht der jeweiligen Formulierung.

Es ist zwar an sich bekannt, daß manche Glycerinester gesättigter Fettsäuren an sich, insbesondere das Glycerinmonolaurat, aber auch die erfindungsgemäß vorteilhaften Glycerinmonocaprylat und Glycerinmonocaprinat, antimikrobielle Wirkung entfalten.

So ist beispielsweise aus der JP-OS Sho-48/019940 der Taiyo Kagaku Kogyo Co. Ltd. ein Kosmetikum mit antiseptischen Eigenschaften bekannt, welches sich durch einen Gehalt an Caprylsäuremonoglycerid auszeichnet.

Schließlich werden in der EP-OS 530 861 topische antimikrobielle pharmazeutische Kombinationen beschrieben, enthaltend neben Glycerinmonocaprylat und Glycerinmonocaprinat auch ein Gemisch aus gesättigten Fettsäuren, welche aber, im Gegensatz zu den Hauptbestandteilen der erfindungsgemäßen Wollwachssäuregemische, unsubstituiert sind und daher keinen Hinweis auf die vorliegende Erfindung geben konnten.

Überraschend war indes, daß die erfindungsgemäßen Wirkstoffgemische sich durch hervorragende Wirkung gegen unreine Haut bzw. Propionibacterium acnes auszeichnen.

Nicht vorhersehbar war nämlich, daß die erfindungsgemäßen Wirkstoffgemische imstande sind, die in tieferen Hautschichten befindlichen, fakultativ anaeroben Propionibacterien überhaupt zu erreichen.

Beim Erscheinungsbild der Akne bzw. unreinen Haut befallen die Propionibacterien zudem die Haarfollikel, welche obendrein von einem dichten Lipidpfropf versiegelt sind. Dies ist, wie der Fachmann weiß, bei vielen potentiell gegen Propionibacterien wirksamen Stoffen das Haupthindernis, gegen Akne bzw. unreine Haut eingesetzt zu werden, da die betreffenden Stoffe geeignete Penetrationseigenschaften eben nicht besitzen.

Als besonders vorteilhafte Verkörperung der vorliegenden Erfindung wird die Verwendung eines Wirkstoffgemisches, bestehend aus
I) einem natürlichen oder durch Destillation erhältlichen Wollwachssäuregemisches und
II) Glycerinmonocaprylat und/oder Glycerinmonocaprinat
zur Bekämpfung des Erscheinungsbildes der unreinen Haut, leichter Formen der Akne sowie Propionibacterium acnes angesehen.

Es ist von Vorteil, den Gehalt an (i) Wollwachssäuregemisch und (ii) Monoglycerin-monocarbonsäure-monoester so zu wählen, daß Verhältnisse (i) : (ii) wie 5 : 1 bis 1 : 20, insbesondere wie etwa 1 : 10, ganz besonders vorteilhaft wie etwa 1 : 5 entstehen.

Es ist insbesondere von Vorteil, den Gehalt an (i) Wollwachssäuren und (ii) Glycerinmonocaprylat und/oder Glycerinmonocaprinat so zu wählen, daß Verhältnisse von (i) : (ii) wie 5 : 1 bis 1 : 20 entstehen. Insbesondere günstig im Sinne der vorliegenden Erfindungen sind Verhältnisse von (i) : (ii) wie 5 : 1 bis 1 : 5, insbesondere wie etwa 1 : 1, ganz besonders vorteilhaft wie etwa 1 : 3.

Erfindungsgemäße Zubereitungen, die erfindungsgemäßen Wirkstoffkombinationen enthaltend, sind besonders vorteilhaft dadurch gekennzeichnet, daß die erfindungsgemäßen Wirksstoffkombinationen in Konzentrationen von 0,05 - 10,00 Gew.-%, bevorzugt 0,1 - 5,0 Gew.-%, vorliegen, jeweils bezogen auf das Gesamtgewicht der Zubereitungen.

Die erfindungsgemäßen gegen unreine Haut wirksamen Zubereitungen können in Form von mittels Pinseln oder Abstreifern oder Roll-on-Vorrichtungen auftragbaren flüssigen Zusammensetzungen, als Stifte und in Form von aus üblichen Flaschen und Behältern auftragbaren W/O- oder O/W-Emulsionen, z.B. Crèmes oder Lotionen. Weiterhin können die erfindinngsgemäßen gegen unreine Haut wirksamen Zubereitungen vorteilhaft in Form von Gesichtswässern, Tinkturen oder Reinigungsformulierungen vorliegen.

Die erfindungsgemäßen gegen unreine Haut wirksamen Zubereitungen können auch in Form von Aerosolen, also aus Aerosolbehältern, Quetschflaschen oder durch eine Pumpvorrichtung versprühbaren Präparaten vorliegen oder in Form von mittels Roll-on-Vorrichtungen auftragbaren flüssigen Zusammensetzungen, als Stifte und in Form von aus normalen Flaschen und Behältern auftragbaren W/O- oder O/W-Emulsionen, z.B. Crèmes oder Lotionen. Ferner ist vorteilhaft, Hydrodispersionen oder Gele als Träger der erfindungsgemäßen Wirkstoffkombinationen zu verwenden.

Weiterhin können die erfindungsgemäßen gegen unreine Haut wirksamen Zubereitungen auch vorteilhaft in Form von Tinkturen, Shampoos, Dusch- oder Badezubereitungen, Pudern oder Pudersprays vorliegen.

Als übliche kosmetische Trägerstoffe zur Herstellung der erfindungsgemäßen Zubereitungen können neben Wasser, Ethanol und Isopropanol, Glycerin und Propylenglykol hautpflegende Fett- oder fettähnliche Stoffe, wie Ölsäuredecylester, Cetylalkohol, Cetylstearylalkohol und 2-Octyldodecanol, in den für solche Präparate üblichen Mengenverhältnissen eingesetzt werden sowie schleimbildende Stoffe und Verdickungsmittel, z.B. Hydroxyethyl- oder Hydroxypropylcellulose, Polyacrylsäure, Polyvinylpyrrolidon, daneben aber auch in kleinen Mengen cyclische Silikonöle (Polydimethylsiloxane) sowie flüssige Polymethylphenylsiloxane niedriger Viskosität.

Als Treibmittel für erfindungsgemäße, aus Aerosolbehältern versprühbare Zubereitungen sind die üblichen bekannten leichtflüchtigen, verflüssigten Treibmittel, beispielsweise Kohlenwasserstoffe (Propan, Butan, Isobutan) geeignet, die allein oder in Mischung miteinander eingesetzt werden können. Auch Druckluft ist vorteilhaft zu verwenden.

Natürlich weiß der Fachmann, daß es an sich nichttoxische Treibgase gibt, die grundsätzlich für die vorliegende Erfindung geeignet wären, auf die aber dennoch wegen bedenklicher Wirkung auf die Umwelt oder sonstiger Begleitumstände verzichtet werden sollte, insbesondere Fluorchlorkohlenwasserstoffe (FCKW).

Als Emulgatoren zur Herstellung der erfindungsgemäßen Zubereitungen, welche vorteilhaft als flüssige Zubereitungen mittels einer Roll-on-Vorrichtung auf die gewünschten Hautbereiche aufgetragen werden sollen, und die in den Zubereitungen in geringer Menge, z.B. 2 bis 5 Gewichts.-%, bezogen auf die Gesamtzusammensetzung, verwendet werden können, haben sich nichtionogene Typen, wie Polyoxyethylen-Fettalkoholether, z.B. Cetostearylalkoholpolyethylenglykolether mit 12 bzw. 20 angelagerten Ethylenoxid-Einheiten pro Molekül, Cetostearylalkohol sowie Sorbitanester und Sorbitanester-Ethylenoxid-Verbindungen (z.B. Sorbitanmonostearat und Polyoxyethylensorbitanmonostearat) und langkettige höhermolekulare wachsartige Polyglykolether als geeignet erwiesen.

Zusätzlich zu den genannten Bestandteilen können den erfindungsgemäßen Zubereitungen gemäß der Erfindung, deren pH-Wert vorzugsweise z.B. durch übliche Puffergemische auf 4,0 bis 9,0 insbesondere 5,0 bis 6,5, eingestellt wird, Parfüm, Farbstoffe, Antioxidantien , Suspendiermittel, Puffergemische oder andere übliche kosmetische Grundstoffe beigemischt werden.

Der pH-Wert der erfindungsgemäßen kosmetischen Zubereitungen wird bevorzugt so eingestellt, daß die erfindungsgemäßen Säurekomponenten im wesentlichen als Säuren, und nicht als Anionen, vorliegen, also bevorzugt im sauren bis neutralen Bereich, insbesondere im pH-Bereich von 5,0 - 6,5.

Erfindungsgemäß können als günstige Antioxidantien alle für kosmetische und/oder dermatologische Anwendungen geeigneten oder gebräuchlichen Antioxidantien verwendet werden.

Vorteilhaft werden die Antioxidantien gewählt aus der Gruppe bestehend aus Aminosäuren (z.B. Glycin, Histidin, Tyrosin, Tryptophan) und deren Derivate, Imidazole (z.B. Urocaninsäure) und deren Derivate, Peptide wie D,L-Carnosin, D-Carnosin, L-Carnosin und deren Derivate (z.B. Anserin), Carotinoide, Carotine (z.B. α-Carotin, β-Carotin, Lycopin) und deren Derivate, Liponsäure und deren Derivate (z.B. Dihydroliponsäure), Aurothioglucose, Propylthiouracil und andere Thiole (z.B. Thioredoxin, Glutathion, Cystein, Cystin, Cystamin und deren Glycosyl-, N-Acetyl-, Methyl-, Ethyl-, Propyl-, Amyl-, Butyl- und Lauryl-, Palmitoyl-, Oleyl-, γ-Linoleyl-, Cholesteryl- und Glycerylester) sowie deren Salze, Dilaurylthiodipropionat, Distearylthiodipropionat, Thiodipropionsäure und deren Derivate (Ester, Ether, Peptide, Lipide, Nukleotide, Nukleoside und Salze) sowie Sulfoximinverbindungen (z.B. Buthioninsulfoximine, Homocysteinsulfoximin, Buthioninsulfone, Penta-, Hexa-, Heptathioninsulfoximin) in sehr geringen verträglichen Dosierungen (z.B. pmol bis µmol/kg), ferner (Metall)-Chelatoren (z.B. α-Hydroxyfettsäuren, Palmitinsäure, Phytinsäure, Lactoferrin), α-Hydroxysäuren (z.B. Citronensäure, Milchsäure, Apfelsäure), Huminsäure, Gallensäure, Gallenextrakte, Bilirubin, Biliverdin, EDTA, EGTA und deren Derivate, ungesättigte Fettsäuren und deren Derivate (z.B. γ-Linolensäure, Linolsäure, Ölsäure), Folsäure und deren Derivate, Ubichinon und Ubichinol und deren Derivate, Vitamin C und Derivate (z.B. Ascorbylpalmitat, Mg-Ascorbylphosphat, Ascorbylacetat), Tocopherole und Derivate (z.B. Vitamin-E-acetat), Vitamin A und Derivate (Vitamin-A-palmitat) sowie Koniferylbenzoat des Benzoeharzes, Rutinsäure und deren Derivate, Ferulasäure und deren Derivate, Butylhydroxytoluol, Butylhydroxyanisol, Nordihydroguajakharzsäure, Nordihydroguajaretsäure, Trihydroxybutyrophenon, Harnsäure und deren Derivate, Mannose und deren Derivate, Zink und dessen Derivate (z.B. ZnO, ZnSO₄) Selen und dessen Derivate (z.B. Selenmethionin), Stilbene und deren Derivate (z.B. Stilbenoxid, Trans-Stilbenoxid) und die erfindungsgemäß geeigneten Derivate (Salze, Ester, Ether, Zucker, Nukleotide, Nukleoside, Peptide und Lipide) dieser genannten Wirkstoffe.

Die Menge der Antioxidantien (eine oder mehrere Verbindungen) in den Zubereitungen beträgt vorzugsweise 0,001 bis 30 Gew.-%, besonders bevorzugt 0,05 - 20 Gew.-%, insbesondere 1 - 10 Gew.-%, bezogen auf das Gesamtgewicht der Zubereitung.

Sofern Vitamin E und/oder dessen Derivate das oder die Antioxidantien darstellen, ist vorteilhaft, deren jeweilige Konzentrationen aus dem Bereich von 0,001 - 10 Gew.-%, bezogen auf das Gesamtgewicht der Formulierung, zu wählen.

Sofern Vitamin A, bzw. Vitamin-A-Derivate, bzw. Carotine bzw. deren Derivate das oder die Antioxidantien darstellen, ist vorteilhaft, deren jeweilige Konzentrationen aus dem Bereich von 0,001 - 10 Gew.-%, bezogen auf das Gesamtgewicht der Formulierung, zu wählen.

Die jeweils einzusetzenden Mengen an kosmetischen bzw. dermatologischen Trägerstoffen und Parfüm können in Abhängigkeit von der Art des jeweiligen Produktes vom Fachmann durch einfaches Ausprobieren leicht ermittelt werden.

Zur Parfümierung, falls gewünscht, sind gegebenenfalls auch solche Substanzen und Parfümöle geeignet, die stabil sind, die Haut nicht reizen und bereits als solche antibakterielle oder bakteriostatische Eigenschaften besitzen.

Die Herstellung der erfindungsgemäßen Zubereitungen erfolgt, abgesehen von speziellen Zubereitungen, die in den Beispielen jeweils gesondert vermerkt sind, in üblicher Weise, zumeist durch einfaches Vermischen unter Rühren, gegebenenfalls unter leichter Erwärmung. Sie bietet keine Schwierigkeiten. Für Emulsionen werden Fettphase und die Wasserphase z.B. separat, gegebenenfalls unter Erwärmen hergestellt und dann emulgiert.

Ansonsten sind die üblichen Maßregeln für das Zusammenstellen von kosmetischen Formulierungen zu beachten, die dem Fachmann geläufig sind.

Sollen die erfindungsgemäßen Zusammensetzungen in Pudersprays eingearbeitet werden, so können die Suspensionsgrundlagen dafür vorteilhaft gewählt werden aus der Gruppe Kieselsäuregele (z.B. solche die unter dem Handelsnamen Aerosil® erhältlich sind), Kieselgur, Talkum, modifizierte Stärke, Titandioxid, Seidenpulver, Nylonpulver, Polyethylenpulver und verwandten Stoffen.

Es folgen vorteilhafte Ausführungsbeispiele der vorliegenden Erfindung. Die angagebenen Zahlenwerte beziehen sich stets auf Gew.-%, sofern nicht ausdrücklich etwas Anderes vermerkt wird. In den Beispielen bedeutet der Begriff "WWS" eine Wollwachssäurefraktion, welche gewonnen wurde aus Rohwollwachssäure durch Kurzwegdestillation im bei 10⁻¹ bar aus dem Destillationstemperaturintervall von 150 - 200° C. Der Anteil an α-Hydroxycarbonsäuren beträgt dabei ca. 22 - 27 %

### Beispiel 1

| Gel Roll-On I - III | | | |
|---|---|---|---|
| | I | II | III |
| PEG-40 Hydriertes Rizinusöl | 1,75 | 1,50 | 1,75 |
| WWS | 0,40 | 0,40 | 0,40 |
| GMC | 0,75 | 0,60 | - |
| GMCy | - | - | 0,90 |
| Ethanol | 62,00 | 62,00 | 60,00 |
| Parfum | q.s. | q.s. | q.s. |
| Wasser, VES | - jeweils ad 100,00 - | | |

### Beispiel 2

| Emulsions Roll-On I - III | | | |
|---|---|---|---|
| | I | II | III |
| PEG-21 Stearylether (Brij 721) | 1,50 | 1,60 | 1,50 |
| PEG-2 Stearylether (Brij 72) | 2,50 | 2,80 | 2,50 |
| Mineralöl DAB 9 | 4,00 | 4,50 | 4,00 |
| Isopropylpalmitat | 3,50 | 3,50 | 4,00 |
| Methyl-Propyl Paraben | 0,15 | 0,15 | 0,15 |
| WWS | 0,70 | 0,70 | 0,70 |
| GMC | 0,70 | - | 0,50 |
| GMCy | - | 0,90 | - |
| Parfum | q.s. | q.s. | q.s. |
| Wasser, VES | - jeweils ad 100,00 - | | |

### Beispiel 3

| Pumpzerstäuber I - II | | |
|---|---|---|
| | I | II |
| Ethanol | 68,00 | 60,00 |
| Propylenglycol-1,2 | 1,80 | 1,80 |
| WWS | 0,30 | 0,30 |
| GMC | 0,70 | - |
| GMCy | - | 1,1 |
| Parfum | q.s. | q.s. |
| Wasser, VES | jeweils ad 100,00 | |

### Beispiel 4

| Gel-Stift I - II | | |
|---|---|---|
| | I | III |
| Stearinsäure | 6,00 | 6,00 |
| Ceteareth-15 | 2,75 | 2,75 |
| WWS | 2,00 | 2,00 |
| GMC | 1,50 | - |
| GMCy | - | 1,60 |
| Ethanol | 16,00 | 16,00 |
| NaOH | 1,10 | 1,05 |
| Parfum | q.s. | q.s. |
| Wasser, VES | - jeweils ad 100,00 - | |

### Beispiel 5

| Deo-Crème I - II | | |
|---|---|---|
| | I | II |
| Mineralöl DAB 9 | 3,50 | 3,50 |
| PEG-40 Stearat | 4,00 | 4,00 |
| Cetylalkohol | 3,50 | 3,50 |
| Ethylhexylstearat | 0,90 | 0,90 |
| Propylenglycol | 1,00 | 1,00 |
| Methyl-propyl-paraben | 0,15 | 0,15 |
| WWS | 0,30 | 0,30 |
| GMC | 0,60 | - |
| GMCy | - | 1,1 |
| Parfum | q.s. | q.s. |
| Wasser, VES | jeweils ad 100,00 | |

## Patentansprüche

1. Verwendung von Wirkstoffkombinationen aus
I) einem natürlichen Wollwachssäuregemisch oder einem durch Destillation eines natürlichen Wollwachssäuregemisches erhältlichen Wollwachssäuregemisch und
II) Monoglycerin-monocarbonsäure-monoestern
gegen unreine Haut, leichte Formen der Akne sowie Propionibacterium acnes.

2. Verwendung nach Anspruch 1, dadurch gekennzeichnet, daß die Wirkstoffkombinationen in kosmetischen oder dermatologischen Zubereitungen eingearbeitet sind.

3. Verwendung nach Anspruch 1, dadurch gekennzeichnet, daß das Wollwachssäuregemisch erhältlich ist aus Rohwollwachssäure durch Kurzwegdestillation im Vakuum bei 10⁻¹ bar aus dem Destillationstemperaturintervall von 150 - 200° C.

4. Verwendung nach Anspruch 1, dadurch gekennzeichnet, daß das durch Destillation erhältliche Wollwachssäuregemisch sich durch folgende Parameter auszeichnet:
| | |
|---|---|
| Tropfpunkt | 50 - 54° C |
| Säurezahl | 166 - 170 |
| Verseifungszahl | 175 - 190 |
| OH - Zahl | 60 - 80 |
| Jodzahl | 7 - 12 |

5. Verwendung nach Anspruch 1, dadurch gekennzeichnet, daß der oder die Monoglycerin-monocarbonsäure-monoester gewählt wird aus der Gruppe Glycerinmonocaprylat und Glycerinmonocaprinat.
